(19) Europäisches Patentamt / European Patent Office / Office européen des brevets

(11) **EP 3 582 342 A1**

(12) **EUROPEAN PATENT APPLICATION**
published in accordance with Art. 153(4) EPC

(43) Date of publication:
**18.12.2019 Bulletin 2019/51**

(51) Int Cl.:
*H01S 3/00* (2006.01)  *A61B 5/00* (2006.01)

(21) Application number: **18751055.7**

(22) Date of filing: **11.02.2018**

(86) International application number:
**PCT/CN2018/076305**

(87) International publication number:
**WO 2018/145659 (16.08.2018 Gazette 2018/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **10.02.2017 CN 201710073746**
**24.03.2017 CN 201710183351**

(71) Applicant: **Peking University**
**Beijing 100871 (CN)**

(72) Inventors:
• **CHEN, Liangyi**
**Beijing 100871 (CN)**

• **ZONG, Weijian**
**Beijing 100871 (CN)**
• **CHENG, Heping**
**Beijing 100871 (CN)**
• **WU, Runlong**
**Beijing 100871 (CN)**
• **LI, Mingli**
**Beijing 100871 (CN)**
• **ZHANG, Yunfeng**
**Beijing 100871 (CN)**
• **WANG, Aimin**
**Beijing 100871 (CN)**

(74) Representative: **de Rooij, Mathieu Julien**
**Bardehle Pagenberg S.L.**
**Avenida Diagonal 420, 1° 1a**
**08037 Barcelona (ES)**

(54) **FEMTO-PULSE LASER MODULATOR AND MINIATURIZED TWO-PHOTO MICROSCOPIC IMAGING DEVICE**

(57) The present invention provides a femtosecond pulse laser modulator and a miniature two-photon microscopic imaging device. The femtosecond pulse laser modulator includes a negative dispersion light path and a positive dispersion light path, wherein the negative dispersion light path includes a laser input fiber, which is configured to transmit laser compensated for through prechirp; the positive dispersion light path is located between a femtosecond pulse laser and the negative dispersion light path, and is configured to compensate for, through prechirp, negative dispersion of the laser produced in the laser input fiber. The femtosecond laser modulator provided in the present invention provides distortion-free transmission for the femtosecond pulse laser with a center wavelength in 920 nm, and effectively excites commonly used biological indicators. The miniature two-photon microscopic imaging device including this femtosecond laser modulator has high speeds in test and application, with high resolution, and is capable of solving the problem of imaging single dendritic spines in a freely behaving animal.

Fig. 4

**Description**

Technical Field

The present invention relates to the technical field of optical imaging, particularly to a femtosecond pulse laser modulator and a miniature two-photon microscopic imaging device.

Background Art

One of the ultimate goals of neuroscience is to decipher basic principles underlying neuronal information processing in freely behaving animals at subcellular, cellular, circuit, and higher levels. In conjunction with fluorescent indicators, light microscopy has become a fundamental research tool in this task, because it allows for direct visualization of neuronal activity at spatiotemporal scales spanning orders of magnitude. Single synapses are the elementary units of information transfer, processing, and storage, and are crucial for comprehending brain functions and disease mechanisms. The postsynaptic structures-dendritic spines are sub-micrometer structures, deeply buried inside brain, that operate at the millisecond scale. Owing to its intrinsic capacity for optical sectioning and deep tissue penetration, multiphoton microscopy has always been the technique of choice for in vivo noninvasive optical brain imaging in the past two decades. Morphological changes of dendritic spines in live bodies, for example, activities of learning and memory neurons, have been able to be resolved with benchtop Two-photon Microscopy (referred to as "TPM" hereinafter for short). Observation of complex dendritic spine activities in head of a live specimen in an awake state can be resolved with a most advanced benchtop multiphoton microscopy equipped with fast image acquisition (with an acquisition frequency>15 Hz), and high excitation and photodetection efficiency. However, as the live specimen is head-fixed all the time, it is physically constrained and experiences emotional stress during the entire experiment, moreover, no prior evidence indicates that neuronal responses to virtual reality and to free exploration are equivalent. More importantly, social behaviors, such as caregiving, mating, and fighting, are incompatible with head fixation.

To meet these challenges, one ideal solution is to develop miniaturized microscopes capable of visualizing the structural and functional dynamics of dendritic spines of the live specimen in a process of free activities over prolonged periods of time. In 2001 Denk and his colleagues built a first miniature two-photon microscopy (with a full English name "micro Two-Photon Microscopy", referred to as "mTPM" hereinafter for short) based on fiber-tip scanning, and then in the following decade, different approaches were subsequently developed by many other research groups. However, there have been no follow-up biological applications of these mTPMs, primarily because of two major limitations. First, no one could image the most widely used fluorescent probes, for example, GCaMPs, because of lack of appropriate optical fibers that efficiently deliver 920-nm femtosecond laser pulses to specimen without distortion. Second, mTPMs usually exhibit a lower resolution than their theoretical resolutions in *in vivo* experiments, presumably because of low sampling rates, aberrations in miniature graded index (referred to as "GRIN" hereinafter for short) lenses, imperfections in miniature optics, and motion artifacts. At present, activity from neuronal structures smaller than dendrites cannot be robustly resolved by mTPMs despite a theoretical lateral resolution of ~1 $\mu$m.

On the other hand, miniature single-photon wide-field microscopes, for example, a microscope developed by Ghosh and his colleagues, have achieved fast acquisition and a large field of view (Field of Vision, referred to as "FOV" hereinafter for short) and are robust against motion artifacts (at neuronal resolution). However, current noninvasive miniature wide-field microscopes can attain only cellular resolution, and image contrast is compromised by the cumulative out-of-focus background signals. Up to now, a new type of mTPM is still to be developed that can provide very good imaging capability and experimental scheme, and is powerful enough to satisfy neuroscientists' requirements for daily experiments.

Therefore, a technical solution is desired to overcome or at least alleviate at least one of the above defects in the prior art.

Summary

An object of the invention is to provide a femtosecond pulse laser modulator and a miniature two-photon microscopic imaging device to overcome or at least alleviate at least one of the above defects in the prior art.

In order to achieve the above object, the present invention provides a femtosecond pulse laser modulator, including a negative dispersion light path and a positive dispersion light path, wherein the negative dispersion light path includes a laser input fiber, which is configured to transmit laser compensated for through prechirp; the positive dispersion light path is located between a femtosecond pulse laser and the negative dispersion light path, and is configured to compensate for, through prechirp, negative dispersion of the laser produced in the laser input fiber.

The present invention further provides a miniature two-photon microscopic imaging device, including:

a femtosecond pulse laser, configured to produce laser with a center wavelength in 920 nm;

a femtosecond pulse laser modulator of any one of the above, configured to receive the laser output by the femtosecond pulse laser, perform pulse amplification, and output the amplified laser to a miniature probe; and

the miniature probe, wherein the miniature probe includes:

a scan imaging portion, configured to receive the laser output by the femtosecond pulse laser modulator, and scan tissues inside a live specimen using the laser, so as to excite a biological indicator inside the live specimen to produce a fluorescence signal; and

a fluorescence output fiber, configured to output the fluorescence signal.

The present invention further provides a live specimen behavior imaging system, including:

the miniature two-photon microscopic imaging device of any one of the above;

a case, configured to provide a restricted space for free movement of the live specimen;

a wiring installation assembly, through which the laser input fiber and the fluorescence output fiber are mounted on the case in a manner of rotating at will with respect to the case; and

a data collecting assembly, configured to gather the fluorescence signal output by the fluorescence output fiber.

The present invention further provides a miniature two-photon microscopic imaging method, characterized by including:

choosing a region to be imaged: fixing a live specimen, and choosing the region to be imaged from the live specimen using a benchtop two-photon microscope;

collecting a fluorescence signal: mounting the miniature probe of the miniature two-photon microscopic imaging device of any one of the above on the live specimen, and releasing the live specimen, so as to collect the fluorescence signal output from the region to be imaged.

The femtosecond laser modulator provided in the present invention provides distortion-free transmission for the femtosecond pulse laser with a center wavelength in 920 nm, and effectively excites commonly used biological indicators. The miniature two-photon microscopic imaging device (referred to as "FIRM-TPM" hereinafter for short) including this femtosecond laser modulator has high speeds in test and application, with high resolution, and is capable of solving the problem of imaging single dendritic spine in a freely behaving animal. In behavioral paradigms involving irregular, vigorous body and head movements (for example, tail suspension, stepping down from an elevated stage, and social interaction), the miniature microscope in the present invention can observe somata, dendrites, and dendritic spines of cortical neurons labeled with GCaMP6f. In summary, the FIRM-TPM is a representative of next generation miniature microscopes, and it meets requirements of high-resolution brain imaging in freely moving animals.

Brief Description of Drawings

FIG. 1 is a structural schematic diagram of a FIRM-TPM of a preferred embodiment provided in the present invention.

FIG. 2 is a schematic diagram showing a state that a miniature probe in FIG. 1 is mounted on a mouse.

FIG. 3 is a schematic diagram of light path principle of the miniature probe in FIG. 1.

FIG. 4 is a structural schematic diagram of a femtosecond pulse laser modulator of a preferred embodiment provided in the present invention.

FIG. 5a is a structural schematic diagram of a live specimen behavior imaging system using a miniature two-photon microscopic imaging device in FIG. 1 according to the present invention.

FIG. 5b is an exploded schematic diagram of a data collecting assembly and a wiring installation assembly in FIG. 5a.

FIG. 6 is a schematic diagram of a light path of an integrated platform of various properties of a benchtop TPM, a miniature wide-field fluorescence microscope, and the FIRM-TPM in a benchtop two-photon mode, wherein this drawing illustrates a structural schematic diagram of the benchtop TPM.

FIG. 7 is a schematic diagram of a light path of the integrated platform in FIG. 6 in a wide-field imaging mode.

FIG. 8 is a schematic diagram of a light path of the integrated platform in FIG. 6 in a FIRM-TPM imaging mode, wherein this drawing illustrates providing the FIRM-TPM in FIG. 1 on a light path between an objective in the benchtop TPM in FIG. 6 and an object plane (a live specimen or a live sample).

FIG. 9a to FIG. 9f show comparison of two-photon excitation efficiency of GCaMP-6f under 800-nm, 920-nm, and 1030-nm excitation.

FIG. 10a shows transmission loss and dispersion parameters of a custom-designed HC-920 fiber, wherein an upper left inset is a photograph of a section of the HC-920, and an upper right inset is an image of 920-nm laser light emitted by the HC-920 after collimation by a doublet lens with a focal length of 3 mm.

FIG. 10b shows autocorrelation distribution showing width of 920-nm femtosecond laser pulses at an exit of 1-m HC-920 at different laser powers.

In FIG. 10c, left-side image: frequency response of x (light gray) and y (black) axes of a MEMS scanner; right-side image: mechanical tilt angle acts as function of a driving voltage for the x (black) and y (light gray) axes of the MEMS scanner.

FIG. 11a to FIG. 11d illustrate dispersion compensation in the femtosecond pulse laser modulator in FIG. 4.

FIG. 12a to FIG. 12e show comparison of dendritic activities of V-1 cortex from a live specimen in a dark environment under a head-fixed condition and under a free moving condition.

FIG. 13a to FIG. 13c show imaging activity in visual cortex of the live specimen during different behavioral paradigms using the live specimen behavior imaging system in FIG. 5.

FIG. 14a to FIG. 14d show performance of the FIRM-TPM compared with the benchtop TPM and a miniature wide-field microscope.

## Detailed Description of Embodiments

In accompanying drawings, the same or similar reference signs represent the same or similar elements or elements having the same or similar functions. Embodiments of the present invention are described in detail below in combination with the accompanying drawings.

In the description of the present invention, orientational or positional relations indicated by terms "center", "longitudinal", "transverse", "front", "rear", "left", "right", "vertical", "horizontal", "top", "bottom", "inner", "outer" etc. are based on orientational or positional relations as shown in the accompanying drawings, merely for facilitating describing the present invention and simplifying the description, rather than indicating or suggesting that related devices or elements have to be in the specific orientation or configured and operated in specific orientation, and therefore, they should not be construed as limiting the scope of protection of the present invention.

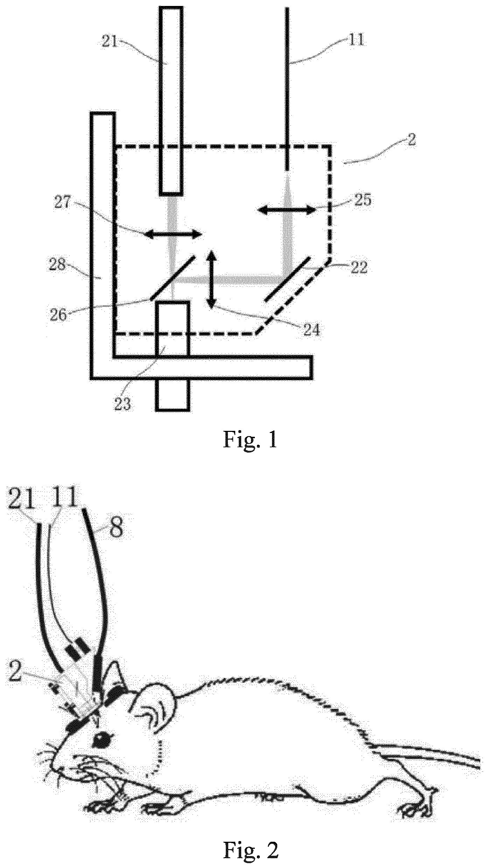

As shown in FIG. 1, a miniature two-photon microscopic imaging device (with a full English name "Fast high resolution miniature two photon microscope", referred to as "FIRM-TPM" hereinafter for short) provided in the present embodiment includes a femtosecond pulse laser (not shown in FIG. 1), a femtosecond pulse laser modulator 1, and a miniature probe 2, wherein

the femtosecond pulse laser is configured to produce laser with a center wavelength in 920 nm. The femtosecond pulse laser, not shown in the drawing, can use a Chameleon solid laser from Coherent Inc. as a light source, with a center wavelength in 920 nm, and an output power of $100\pm1$ mW. Compared with existing laser with a wavelength of 1030 nm and 800 nm, the laser with the wavelength of 920 nm can effectively excite commonly used biological indicators (for example, Thy1-GFP and GCaMP-6f, among which GCaMP is a most generally used calcium ion indicator) to produce fluorescence. Two-photon excitation efficiencies of GCaMP-6f under 800-nm, 920-nm, and 1030-nm laser excitation are compared below. A tunable titanium-sapphire femtosecond laser (Coherent, USA, Vision-S, 680-1080 nm, 85-110 fs) is employed to select different wavelengths for excitation.

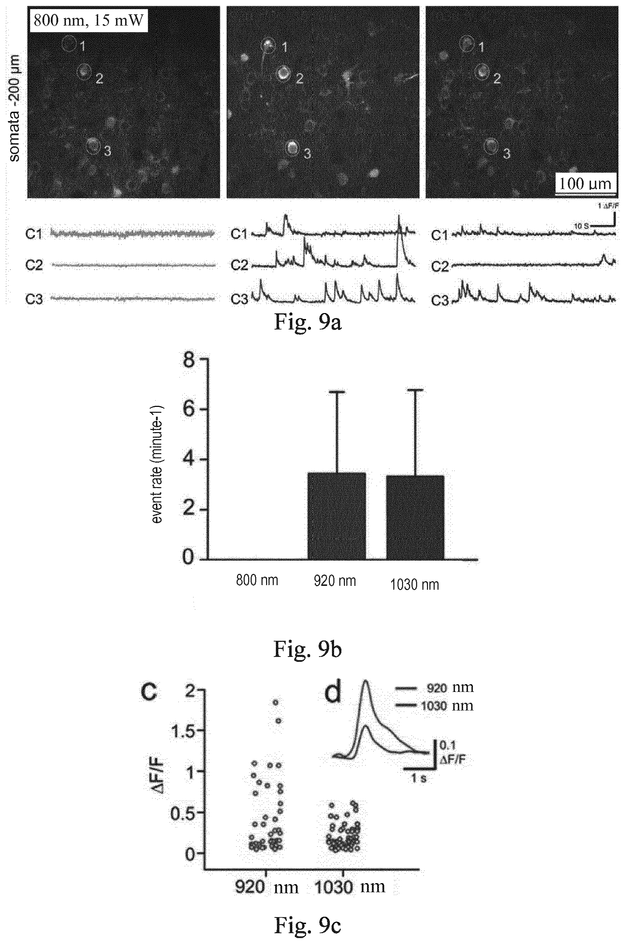

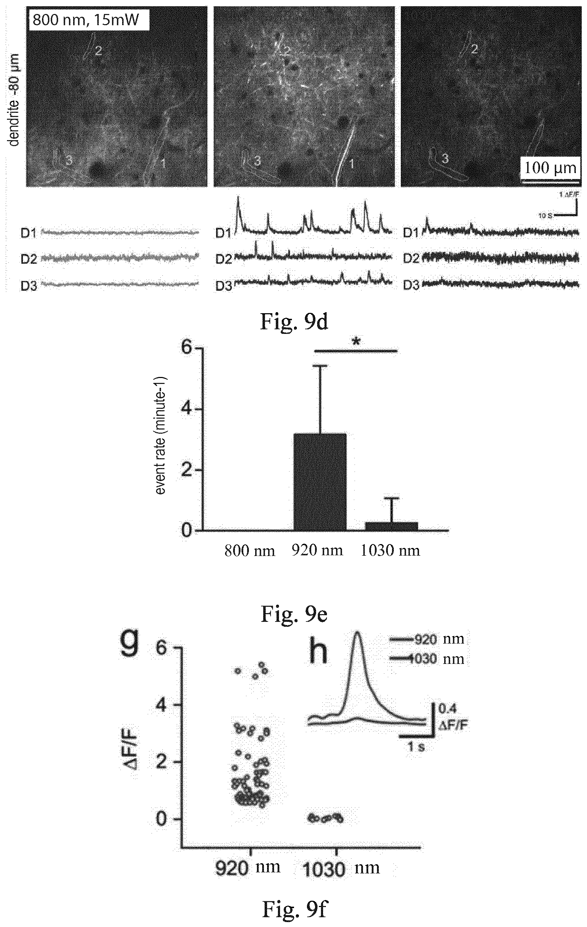

As shown in FIG. 9a to FIG. 9f, FIG. 9a shows imaging of neuronal somata cell 1, cell 2, and cell 3 in a same group in an AAV-transfected live specimen expressing GCaMP-6f in V-1 cortex under different excitation wavelengths, and in the drawing, from left to right, there are fluorescence images obtained using 800-nm, 920-nm, and 1030-nm laser, respectively. FIG. 9b shows frequencies of neuronal somata of $Ca^{2+}$ signals of 10 glia cells. In FIG. 9c, c represents magnitude of the neuronal somata of the $Ca^{2+}$ signals of the 10 glia cells, and d in FIG. 9c represents average time course of the neuronal somata of the $Ca^{2+}$ signals of the 10 glia cells. For FIG. 9d, as in FIG. 9a, neuronal dendrites, dendrite 1, dendrite 2, and dendrite 3, are imaged on different focal planes, and in the drawing, from left to right, there are fluorescence images obtained using 800-nm, 920-nm, and 1030-nm laser, respectively. FIG. 9e shows frequencies of 10 dendritic $Ca^{2+}$ signals shown in FIG. 9d. In FIG. 9f, g represents magnitude of the 10 dendritic $Ca^{2+}$ signals shown in FIG. 9d, and h in FIG. 9f represents average time course of the 10 dendritic $Ca^{2+}$ signals shown in FIG. 9d. As can be seen from the experimental data: the 800-nm-centered femtosecond laser pulse is completely unable to excite a GCaMP6 signal from the neuronal somata or dendrites, and compared with 920-nm excitation, 1030-nm excitation merely can record 1/10 of activity of the dendritic calcium ion, meanwhile, the recorded fluorescence signal has remarkably reduced magnitude. Experimental data further proves that use of the laser with a wavelength of 920 nm can more effectively excite GCaMP-6f to produce fluorescence.

Referring to FIG. 1 again, the femtosecond pulse laser modulator 1 is configured to receive laser output by the femtosecond pulse laser, amplify a transmission power of the laser to a predetermined value, compensate for pulse-broadening of the laser through prechirp, and then output the compensated laser. For example, the 85-fs, 920-nm-centered laser pulse, after amplification by the femtosecond pulse laser modulator 1, is modulated into laser pulse of approximately 100 fs, and the transmission power can vary from 5 mW to 200 mW (the data is measured at an exit of a laser input fiber 11 in an experiment).

The miniature probe 2 includes a scan imaging portion and a fluorescence output fiber 21, wherein the scan imaging portion is configured to receive the laser output by the femtosecond pulse laser modulator 1, which laser scans tissues inside a live specimen so as to excite the live specimen to produce a fluorescence signal. The fluorescence output fiber 21 is configured to receive the fluorescence signal output by the scan imaging portion, and output the fluorescence signal.

As shown in FIG. 1 and FIG. 2, a new type of supple fiber bundle (SFB) is chosen as the fluorescence output fiber 21. The fluorescence output fiber 21 in the present embodiment is made by fusing 700-900 glass optical fibers while keeping various glass optical fibers loose and separated in-between, thus it is easy for the freely behaving live specimen to carry a light microscope probe with reduced motion artifacts, further minimizing torque and tension caused by animal movement, without decreasing photon collection efficiency. Both fused ends of each of all the glass optical fibers form a 1.5-mm-diameter cylinder, so as to be easily mounted on the miniature probe 2. Following Table 1 shows property parameters of the fluorescence output fiber 21 in the present embodiment. The fluorescence output fiber has higher collection efficiency as compared to multimode fibers, is more flexible than plastic fibers and traditional fusion-type fiber bundles used previously, and is capable of multiwavelength emission detection. The fluorescence output fiber 21 delivers the fluorescence signal from an objective in the miniature probe 2 to a distal GaAsP PMT (10770P-40, Hamamatsu, Japan), with total collection efficiency of approximately 80% at 532 nm.

Table 1

| Quantity of Fibers | 800 |
|---|---|
| Diameter of Single Fiber ($\mu$m) | 30 |
| Numerical Aperture (NA) | 0.65 |
| Collection Angle (degree) | 80 |
| Transmission Efficiency (%/m) | 90 |
| Duty Cycle (%) | 90 |
| Overall Transmission Efficiency (%) | 80 |

As shown in FIG. 1 and FIG. 2, the miniature probe 2 is integrated on a fixed holder 28. Through the fixed holder 28, the miniature probe 2 is detachably (screw connection) mounted on head of the live specimen (such as a mouse shown in FIG. 2 and the like). The miniature probe 2 and the fixed holder 28 weigh about 2.15 g in total, and occupy no more than 1 cm$^3$, hence sufficiently small and compact and light to be easily borne by the live specimen. The fixed holder 28 is made by an aluminum material, for example, an aluminum frame. The fixed holder 28 may be designed in a helmet shape, thus it is easy to mount and detach, and importantly, it can solidly mount the miniature probe 2 on the head of the freely behaving live specimen, therefore, it is favorable to hours of imaging for a same animal, and guarantees no drift of field of vision (referred to as "FOV" hereinafter for short) of a miniature two-photon microscopic imaging device in cases of intense body and head movements. Besides, before imaging with the present invention, the live specimen is trained in advance to be adapted to the miniature probe mounted on skull thereof and 1.5% low-melting-point agarose is added dropwise to fill in between the objective of the miniature probe 2 and brain tissues of the live specimen. All of these operations significantly dampen relative movements between the miniature probe 2 and the brain. These measures improve short- and long-term stability of experiments, allowing for high-resolution imaging in detection of animals' behaviors involving lots of free activities of the body and head.

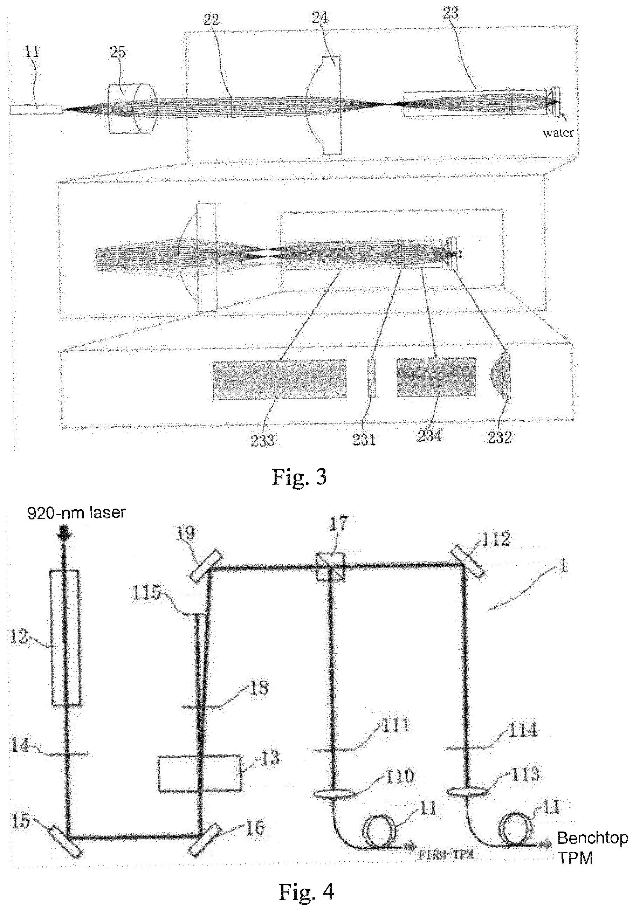

In one embodiment, as shown in FIG. 4, the femtosecond pulse laser modulator 1 has a negative dispersion light path and a positive dispersion light path, wherein the negative dispersion light path includes the laser input fiber 11, and the laser input fiber 11 is configured to transmit the laser already compensated for in the pulse-broadening through prechirp to the scan imaging portion, and the positive dispersion light path is located between the femtosecond pulse laser and the negative dispersion light path, and is configured to compensate for negative dispersion caused by the laser input fiber 11 in the process of laser transmission.

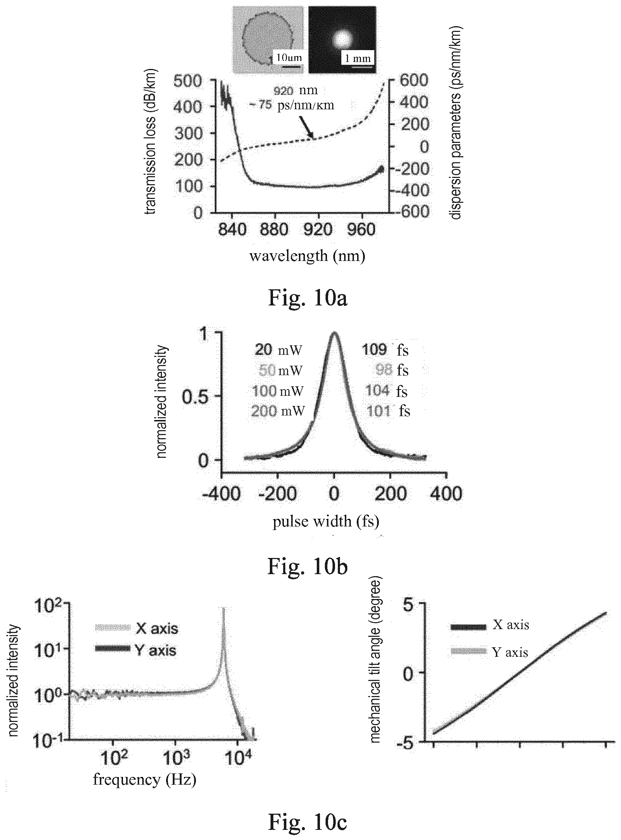

A single-mode optical fiber broadens the femtosecond laser pulse because of material dispersion and nonlinear effects. However, the laser input fiber 11 used in the present embodiment is a hollow-core photonic crystal fiber (referred to as "HC-PCF" hereinafter for short), i.e. HC-920 appearing in the drawings or the text, wherein most of the laser light travels through an air-filled core within the HC-920, which effectively minimizes the pulse-broadening due to the nonlinear effects. The laser input fiber 11 in the present embodiment is configured to transmit hundreds of milliwatts of 920-nm femtosecond laser pulses, with negligible nonlinear pulse-broadening thereof (FIG. 10a and Table 2).

Table 2

| HC-920 | |
|---|---|
| Material | |
| Fiber material: | Pollution-free silicon |
| Coating material: | Acrylate (single layer) |
| | |
| Dimensions | |
| Fiber core diameter: | 8-9 $\mu$m |
| Cladding diameter: | 152 $\mu$m |
| Coating diameter: | 255 $\mu$m |
| Optical indices | |
| Mode field diameter r under 900-nm wavelength | 7 $\mu$m |
| Unpolarized transmission loss under 920-nm wavelength | 100 dB/km |
| Group dispersion under 920-nm wavelength | 75 ps/nm/km |
| Measured transmission efficiency for 1 m under 920-nm wavelength | 92 $\pm$ 2% |

The laser input fiber 11 in the present embodiment provides distortion-free transmission for the 920-nm femtosecond laser pulse. Such improvement allows it possible to effectively excite commonly used biological indicators, for example, Thy1-GFP and GCaMP-6f.

In one embodiment, the positive dispersion light path includes a dispersion compensation element 12 and an acousto-optic modulator 13, wherein the acousto-optic modulator 13 is configured to receive laser compensated for by the dispersion compensation element 12, adjust laser intensity, and then output the laser to the scan imaging portion. The acousto-optic modulator 13 may be implemented using an existing product.

The dispersion compensation element 12 is provided close to the femtosecond pulse laser, and is configured to compensate for negative dispersion caused by the laser input fiber 11 in the process of laser transmission.

The negative dispersion in the laser input fiber 11 is relatively low, and can be accurately compensated for by a prechirp method provided that the dispersion compensation element 12 uses a commercial material with positive dispersion of a specific length. The commercial material herein refers to universal material with respect to custom-made laser input fiber 11, that is, after the negative dispersion of the laser input fiber is determined, an appropriate positive dispersion compensation element, i.e. the dispersion compensation element 12 is found by selecting the material and length. After the prechirp compensation, the 85-fs, 920-nm-centered laser pulses, after being transmitted by the 1-m-long laser input fiber 11, becomes pulse of about 100 fs, and the transmission power can vary from 5 mW to 200 mW (the data is measured at the exit of the laser input fiber 11 in an experiment).

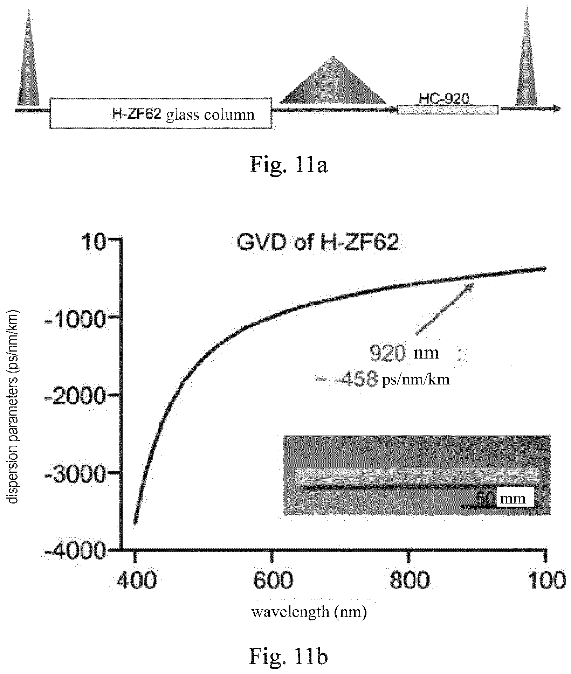

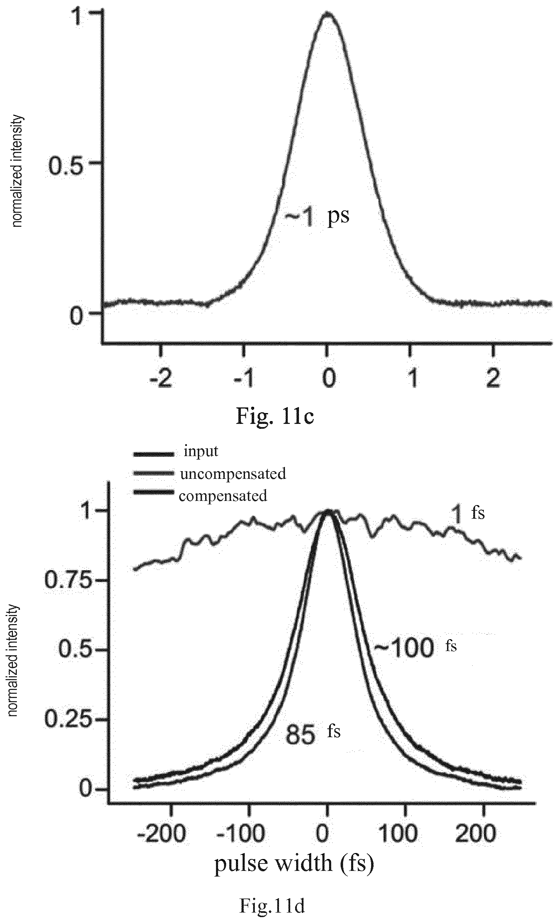

Preferably, an H-ZF62 glass tube is used as the dispersion compensation element 12, which has positive dispersion of 458 ps/nm/km in laser with a wavelength of 920 nm, wherein FIG. 11a is a schematic diagram showing compensation of dispersion of the HC-920 using the H-ZF62 glass tube; FIG. 11b shows dispersion parameters of glass H-ZF62; FIG. 11c shows pulse width of 920-nm femtosecond laser light output from the 1-m HC-920 without dispersion compensation; FIG. 11d shows pulse width of 920-nm femtosecond laser from the 1-m HC-920, and pulse widths of 920-nm femtosecond laser from the 1-m HC-920 before and dispersion compensation.

It is described in detail below that after prechirp compensation, the 85-fs, 920-nm-centered laser pulses are converted into laser pulses of ~100 fs by the femtosecond pulse laser modulator 1, and the transmission power can vary from 5 mW to 200 mW.

During transmission of ultrafast laser pulse, two factors lead to pulse-broadening: material dispersion and nonlinear effects of a high laser peak power. In the laser input fiber 11, most of the laser is propagated through an air core, thus the nonlinear effects are minimal. In a light path of the miniature probe 2, an optical part such as lens and reflector has relatively low optical density at center, therefore, a beam dimension is relatively large (with a diameter of no more than 2 mm), and thus almost no nonlinearity is induced. Since a front part of the microscope and a miniature part in the specimen are relatively short in length, they are also negligible. Therefore, a main spreading effect originates from material dispersion caused by the benchtop optics and the laser input fiber. Through simplification, only second-order dispersion is taken into consideration in the system of the present embodiment, therefore, the pulse-broadening of the whole system is:

$$Bsystem=|\sum n\{Ln\int\lambda,\Delta\lambda Dn(\lambda)d\lambda\}|(1.1),$$

where n represents the number of optics in the system; $Dn(\lambda)$ represents a dispersion parameter induced by an n-th component (optics); Ln represents a light path length of the n-th component (optics); $\lambda$ and $\Delta\lambda$ represent center wavelength and spectrum width of laser; | | represents an absolute value of a sum of positive dispersion (negative $D(\lambda)$) from the lens and abnormal dispersion (positive $D(\lambda)$) from the laser input fiber. Therefore, when $\Delta\lambda<<\lambda$, in the system of the present invention ($\lambda=920$ nm, and $\Delta\lambda$ is no more than 15 nm), the equation is simplified as follows:

$$Bsystem=|\sum n\{Ln\cdot Dn(\lambda)\cdot\Delta\lambda\}|(1.2)$$

A final pulse width is represented by:

$$Poutput=Pinput+|Bsystem|(1.3)$$

where Pinput and Poutput represent input pulse width and output pulse width of the laser.

The HC-920 used in the system provided in the present embodiment has a length of 1 m (Lhc-920), measured by a manufacturer and shown in FIG. 10a. Therefore, the HC-920 is expanded as:

$$Lhc\text{-}920\cdot Dhc\text{-}920(920nm)\cdot\Delta\lambda\sim1(m)\cdot75(ps/nm/km)\cdot15(nm)=1.125(ps)$$

Lengths of such as a polarization beam splitter, the lens, and the acousto-optic modulator are easily determined, and calculated through information of refractive indices of optic they use, which is represented by the following:

$$GVD = \frac{\lambda^3}{2\pi c^3}\left(\frac{d^2 n}{d\lambda^2}\right) \qquad (1.4)$$

and

$$D(\lambda) = -\frac{2\pi c}{\lambda^2}\cdot GVD \qquad (1.5)$$

where GVD represents group velocity dispersion in a medium, c is velocity of light, and n is refractive index of the medium.

These components add up to cause broadening of approximately -200 fs, therefore, calculated pulse width output from the 85-fs HC-920 of transform limited pulse is approximately as follows:

$$(0.085+1.125-0.2)(ps)=1.01(ps)$$

It is matched with the pulse width (no more than 1 ps) (FIG. 11a) measured in the experiment, and is too weak for effective two-photon excitation. By inserting the H-ZF62 glass tube (the H-ZF62 glass tube is used as the dispersion compensation element 12 in the present embodiment) into the light path with positive dispersion, the negative dispersion induced by the HC-920 is compensated for in the present embodiment. The H-ZF62 glass tube is chosen in the present embodiment for the reason that it has large positive dispersion (the dispersion is approximately 458 ps/nm/km in a 920-nm wavelength), calculated by the refractive index information supplied by Scott company and equation (1.4), as shown in FIG. 11b). A desired length of the H-ZF62 glass tube is calculated as follows:

$$(1.125-0.2)(ps)/458(ps/nm/km)/15(nm)=13.5(cm)$$

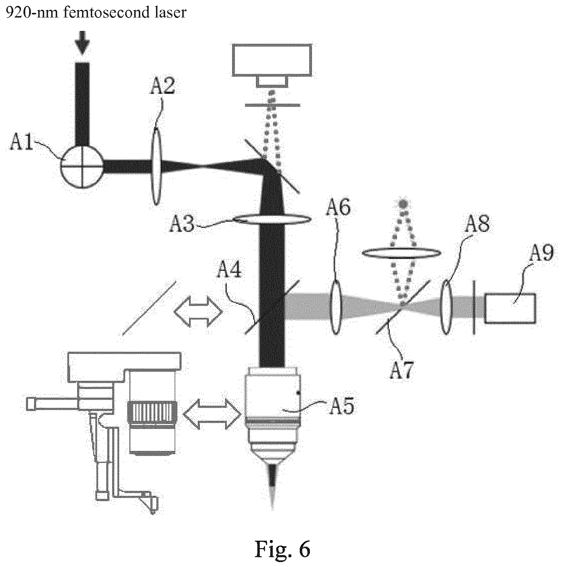

Considering the small positive dispersion induced by a microscope front end and the specimen, the H-ZF62 glass tube having a length of 12 cm and a diameter of 12.7 mm, less than the calculation values, is used in the present embodiment. After such "pre-linear frequency modulation" of the H-ZF62 glass tube (FIG. 11b and FIG. 6), the width of the laser pulse coming from the HC-920 is compressed to ~100 fs within the whole range of illumination power tested (FIG. 11d and FIG. 10b). By using a custom-designed HC-PCF to transmit the 920-nm femtosecond laser pulses (termed HC-920) with negligible nonlinear pulse-broadening, the FIRM-TPM in the present embodiment is able to experimentally image commonly used biological indicators with a performance comparable to that of a benchtop TPM.

In one embodiment, the positive dispersion light path further includes a laser orientation adjustment assembly, wherein the laser orientation adjustment assembly is provided between the dispersion compensation element 12 and the acousto-optic modulator 13, and the laser orientation adjustment assembly includes a first half-wave plate 14, a first reflector 15, and a second reflector 16, wherein
the first half-wave plate 14 is configured to receive the laser compensated for by the dispersion compensation element 12, and adjust a laser polarization direction, such that the acousto-optic modulator 13 reaches highest modulation efficiency. The first reflector 15 is configured to receive the laser passing through the first half-wave plate 14, and reflect the laser, so as to adjust a position where the laser enters the acousto-optic modulator 13. The second reflector 16 is configured to receive the laser passing through the first reflector 15, and reflect the laser, so as to adjust an angle at which the laser enters the acousto-optic modulator 13, and transmit the laser to the acousto-optic modulator 13.

In one embodiment, the positive dispersion light path further includes a spectroscopic assembly, wherein the spectroscopic assembly is provided close to the laser input fiber 11, and is configured to receive the laser whose intensity has been already adjusted by the acousto-optic modulator 13 and split the laser into at least two bundles, and transmit the laser to the laser input fiber 11.

In one embodiment, the laser input fiber 11 is at least two in number, including a first laser input fiber and a second laser input fiber.

The spectroscopic assembly includes a polarization beam splitter 17, a second half-wave plate 18, a third reflector 19, a first collimation lens 110, a third half-wave plate 111, a fourth reflector 112, a second collimation lens 113, a fourth half-wave plate 114, wherein
the polarization beam splitter 17 is configured to receive the laser whose intensity has already been adjusted by the acousto-optic modulator 13 and split the laser into at least two bundles respectively transmitted to the first laser input fiber and the second laser input fiber. The second half-wave plate 18 is arranged between the polarization beam splitter 17 and the acousto-optic modulator 13, and is configured to alter a spectroscopic ratio of the polarization beam splitter 17. The third reflector 19 is arranged between the polarization beam splitter 17 and the second half-wave plate 18, and is configured to reflect the laser, so as to adjust the position of the laser and project the laser onto an incident surface of the polarization beam splitter 17. The first collimation lens 110 is arranged between a first emergent surface of the polarization beam splitter 17 and the first laser input fiber 11, and is configured to receive the laser output by the polarization beam splitter 17 and couple the laser into the first laser input fiber 11. The third half-wave plate 111 is arranged between the first emergent surface of the polarization beam splitter 17 and the first collimation lens 110, and is configured to receive the laser output by the polarization beam splitter 17, and adjust a laser polarization direction, such that the highest efficiency of coupling the laser into the first laser input fiber 11 is reached. The fourth reflector 112 is arranged between a second emergent surface of the polarization beam splitter 17 and the second laser input fiber, and is configured to receive the laser output by the polarization beam splitter 17, and is configured to reflect the laser, so as to adjust the position of the laser and project the laser into the second laser input fiber. The second collimation lens 113 is arranged between the fourth reflector 112 and the second laser input fiber, and is configured to receive the laser output by the polarization beam splitter 17 and couple the laser into the second laser input fiber. The fourth half-wave plate 114 is arranged between the fourth reflector 112 and the second collimation lens 113, and is configured to receive the laser reflected by the fourth reflector 112, and adjust the laser polarization direction, such that the highest efficiency of coupling the laser into the second laser input fiber is reached. A stray light shielding device 115 is optional to avoid emergence of undesired light.

As shown in FIG. 1 and FIG. 3, in one embodiment, the scan imaging portion includes a micro-electromechanical scanner 22, an objective 23, a scan lens 24, a collimator 25, a dichroic mirror 26, and a collecting lens 27, wherein
the micro-electromechanical scanner (MEMS) 22 is configured to perform two-dimensional scanning for a tissue plane inside the live specimen using the laser in a manner of altering magnitude of a laser incident angle through rotation. Preferably, the micro-electromechanical scanner 22 is 0.8 mm in diameter, and has a packaging size of 9x9 mm$^2$. Its first resonant frequency is no more than 6 kHz, and its maximal optical scanning angle is $\pm 10$ degrees. It supports 40-Hz imaging with a frame size of 256x256 and a maximal field of vision of $130 \times 130$ $\mu$m$^2$, so as to realize video-rate image acquisition. Compared with other scanning manners, for example, optical fiber scanning using spiral or Lissajous pattern of a piezoelectric actuator, the scanning of the micro-electromechanical scanner 22 provided in the present embodiment is advantageous to high velocity thereof on the whole field of vision, even excitation and large scanning angle, and large visual field. Control signals x and y in the scanning process of the micro-electromechanical scanner 22 are generated with an FPGA (Field Programmable Gate Array) card (PXI-7853R), which is also used in driving the acousto-optic modulator so as to adjust the laser intensity and in triggering other devices (for example, infrared camera collection). A signal from a photodetector (H10770P-40, Hamamatsu Japan) is amplified with a high-speed preamplifier (DHPCA-100, FEMTO Gmbh, Berlin, Germany), then connected to a 120 MS/s Digitizer Adaptor Module (NI5734, National Instruments Inc., Austin, TX, USA) and a FlexRIO FPGA (PXIe-7961R, National Instruments Inc., Austin, TX, USA) for high-speed data acquisition. The entire system is controlled by custom-developed software based on the LabVIEW platform.

The objective 23 is configured to converge the laser from the micro-electromechanical scanner 22 into the live specimen, so as to excite the live specimen to produce the fluorescence signal and is configured to output the fluorescence signal. Preferably, the numerical aperture (referred to as "NA" hereinafter for short) of the objective 23 is 0.8, a relatively high NA resulting in relatively thin optical sectioning, which is favorable to achieve sub-micrometer imaging resolution, and is further capable of distinguishing structural and functional changes of dendrites and dendritic spines in the freely behaving specimen.

In the present embodiment, a high-resolution water-immersion miniature objective (GT-MO-080-018-AC900-450) from GRINTECH GmbH (Jena, Germany) is used as the objective 23, which is finite-corrected with a magnification of 4.76x, an object NA of 0.80, and an image NA of 0.173. The objective 23 is 6 mm long, 1.4 mm in diameter, and has a working distance of 200 $\mu$m. The objective 23 specifically includes a thin Fresnel lens 231, a plano-convex lens 232, a relay lens 233, and a focus lens 234, wherein the thin Fresnel lens 231 and the plano-convex lens 232 are both miniature graded-index (GRIN) lenses. Compared to the objectives used in the prior art, the diffraction component (Fresnel lens) incident into the objective 23 in the present embodiment efficiently corrects large chromatic aberration between excitation and emission wavelengths during two-photon imaging, which results in better beam focusing quality and improved signal collection efficiency, thus it is advantageous to achieve the sub-micrometer imaging resolution, and further provides an advantageous condition for distinguishing structural and functional changes of the dendrites and the dendritic spines in the freely behaving specimen.

The scan lens 24 is arranged on a light path between the micro-electromechanical scanner 22 and the objective 23, and is configured to convert the laser altered in angle caused by the two-dimensional scanning of the micro-electromechanical scanner 22 into position-altered laser. An aspheric lens (#355160B, Lightpath Technologies, Orlando, FL, USA; diameter: 3 mm; equivalent focal length: 2.7 mm) is used as the scan lens 24 to reduce spherical aberration.

The collimator 25 is arranged between the laser input fiber 11 and the micro-electromechanical scanner 22, and is configured to collimate the laser output by the laser input fiber 11 and reduce chromatic aberration between lasers with different frequencies, so as to match the image NA of the objective 23 together with the scan lens 24. An achromatic collimating lens (#65-286, Edmund Optics Inc., Barrington, NJ, USA; diameter: 2 mm, equivalent focal length: 3 mm, specifically designed for near-infrared light) is used as the collimator 25 in the present embodiment, which can collimate the output laser and reduce the chromatic aberration between different frequency components of the femtosecond laser, which facilitates improvements on the transmission throughput (up to 50% from a laser source to the specimen), beam focusing, and excitation efficiency.

The dichroic mirror 26 is provided between the scan lens 24 and the objective 23, and is configured to separate the laser from the fluorescence signal and output the fluorescence signal. A product produced and manufactured by Sunlight Ltd., Fujian, China is used as the dichroic mirror 26, sized $3 \times 3 \times 0.2$ mm$^3$, with a reflection band of 750-1100 nm, and a transmission band of 400-650 nm, to reflect the laser beam from the scan lens 24 to the objective 23. The collecting lens 27 is configured to effectively collect the fluorescence signal.

Optical-design software (ZEMAX) is used in the present invention to simulate and optimize all optical components and distances between them, and commercial software CAD (Solidworks) is used for geometrical design.

A specific calculation process of the resolution of the miniature probe 2 provided in the present invention is as follows:

Firstly, effective excitation NA (NAex) is determined by the entire light path of excitation. The laser beam behind the collimator 25 has a diameter $D_1$ as follows:

$$D_1 = 2 \cdot NA_{hc\text{-}920} \cdot EFL_c \quad (2.1)$$

where $NA_{hc\text{-}920}$ is NA (approximately 0.15) of the HC-920, and EFLc is an equivalent focal length (3 mm) of the collimator 25.

Since the diameter $D_1$ (0.9 mm) of the laser beam is greater than the size of the micro-electromechanical scanner 22 (0.8 mm in a direction x, and about 0.6 mm in a direction y), considering that a tilt angle of the micro-electromechanical scanner 22 is restricted by the size thereof and given by the following formula:

$$NA_{ex}=(1/2 \cdot D_m/EFL_s) \cdot NA_o/NA_i \quad (2.2)$$

where $EFL_s$ is an equivalent focal length (2.7 mm) of the scan lens 24, $NA_o$ and $NAi$ respectively represent the object NA of the objective and the image NA, being 0.8 and 0.173 respectively, then a maximal excitation NA determined by the direction x of the micro-electromechanical scanner 22 is:

$$NA_{ex}=(1/2 \cdot 0.8/2.7) \cdot 0.8/0.173 \approx 0.685$$

A lateral diameter ($\omega_{xy}$) and an axial diameter ($\omega_z$)1/e of a diffraction limit of point spread function of two-photon excitation (IPSF2) are calculated using equations in Reference Document 2, and in the present embodiment, there are:

$$\omega_{xy} = 0.320 \cdot \lambda / (\sqrt{2} NA_{ex}) \qquad (2.3)$$

$$\omega_z = 0.532 \cdot \lambda / \sqrt{2} \cdot \left[ 1/(n - \sqrt{n^2 - NA_{ex}^2}) \right] \qquad (2.4)$$

where a refractive index of an immersion medium (normal saline) is no more than 1.34, and the wavelength of the laser is 920 nm. $NA_{ex}$ is 0.685, $\omega_{xy}$=0.304 $\mu$m, and $\omega_z$=1.840 $\mu$m.

The resolution is generally defined as full width half maximum (FWHM) of PSF2, and in the present embodiment there are:

$$FWHM_{xy} = 2\sqrt{\ln 2} \cdot \omega_{xy} = 2\sqrt{\ln 2} \cdot 0.304(\mu m) \sim 0.506(\mu m)$$

$$FWHM_z = 2\sqrt{\ln 2} \cdot \omega_z = 2\sqrt{\ln 2} \cdot 1.840(\mu m) \sim 3.063(\mu m)$$

It is obtained that the miniature two-photon microscopic imaging device provided in the present invention has a lateral resolution of 0.64±0.02 $\mu$m, and an axial resolution of 3.35±0.36 $\mu$m as measured from images of 100-nm fluorescence beads embedded in agarose.

In the above embodiment, a method of preparing an experimental specimen when testing the resolution in the present invention includes: first 2 $\mu$L of TetraSpeck 100-nm fluorescent beads (T7270, Life Technologies, Oregon USA) is diluted into 100 $\mu$L of normal saline, then mixed with 900 $\mu$L 1.5% low-melting-point agarose. A droplet of the agarose containing the microspheric fluorescent beads (4 $\mu$M) is applied to a coverglass, and the specimen is ready for use after 10 minutes. An equivalent lateral pixel size is 61 nm, and an axial scan interval is 80 nm, both of which are more than 5 times above the Nyquist requirement are sufficient to measure a real optical resolution of the microscope.

Table 3 lists comparison of resolutions of the FIRM-TPM provided in the present invention to the mTPMs in the prior art, where column 2 lists the resolution of the FIRM-TPM provided in the present invention and parameter explanation thereof, and column 3 to column 6 list the resolutions of the mTPMs in the prior art and parameter explanation thereof. It should be indicated that * in Table 3 means that the data comes from reference document. Existing experimental data prove that although the mTPMs in the prior art render a relatively high spatial resolution outside animal bodies, most of the mTPMs do poorly within the animal bodies, and fail to provide a fluorescence image with a high signal-to-noise ratio (SNR). As can be seen from comparative analysis in Table 3, the resolution of the FIRM-TPM provided in the present invention is roughly double the highest resolution of the mTPMs in the prior art, which high resolution provides an advantageous condition for solving the problem of imaging single dendritic spines in the freely behaving animals.

Table 3

| | FIRM-TPM | Denk Research Group 2001 (31) | Helmche Research Group 2008 (32) | Kerr Research Group 2009 (33) | Schnitzer Research Group 2009 (34) |
|---|---|---|---|---|---|
| Resolution | lateral: 0.65 $\mu$m axial: 3.3 $\mu$m | lateral: unknown axial: unknown | lateral: 0.98 $\mu$m axial: 7.68 $\mu$m | lateral: 1 $\mu$m axial: unknown | lateral: 1.29 $\mu$m axial: 10. 3 $\mu$m |
| Maximal FOV | 130×13$\mu$m$^2$ | 65×65$\mu$m$^2$ | 200 × 200 $\mu$ m$^2$ | ~250×50$\mu$m$^2$ * | ~100×95$\mu$m$^2$ |
| Highest Frame Rate | 40 Hz 256×256 | 2 Hz 128×128 | 8 Hz 512×512 | 8.2 Hz 64×64 | 4 Hz 400×135 |
| Scan Mechanism | MEMS scan | optical fiber scan | optical fiber scan | optical fiber scan | MEMS scan |
| Weight | 2.15 g | 25 g | 0.6 g | 5.5 g | 2.9 g |
| Excitation Wavelength | 920 nm | 820 - 850 nm | 812 nm | 925 nm | 790 - 810 nm |
| Pulse Width behind Objective | approxmately 100 fs | 100 fs 10 mW 1 ps 180 mW | 100 fs | 1.8 ps | ~110 fs |
| Power behind Objective | 10-25 mW | unstated | approxmately 150-200 mW | 100-150 mW | 27 mW |
| Free-moving Experiment | live specimen | rat | unspecified | rat | unspecified |
| GCaMP Imaging | possible | impossible | impossible | quite low efficiency | impossible |

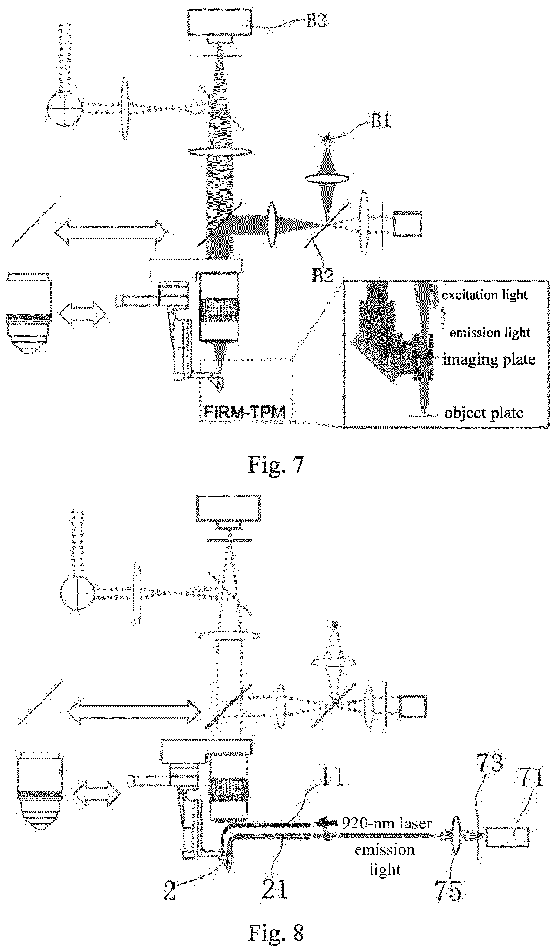

In order to test and compare performance differences of the FIRM-TPM with the benchtop TPMs as well as the miniature wide-field microcopy in the prior art in an unbiased manner, an integrated platform that allows for imaging of the same specimen during switching among a benchtop TPM mode (FIG. 6), a wide-field imaging mode (FIG. 7), and the FIRM-TPM imaging mode (FIG. 8) is built in the present embodiment. As shown in FIG. 6 to FIG. 8, the integrated platform is equipped with miniature and conventional objectives, multiple illumination sources (lasers for TPMs and for a high-power mercury lamp for wide-field microscopy), and detecting devices (GaAsP for TPMs and a sCMOS camera for wide-field microscopy). Different imaging modes share the same focal plane and concentric FOVs, total frame-acquisition time, and imaging NA, whereas the average illumination power and detector sensitivity are also carefully selected for comparison. The composition of the above integrated platform is described below in combination with FIG. 6 to FIG. 8.

The above integrated platform images the same focal plane of the same sample in the benchtop TPM mode, the wide-field imaging mode, and the FIRM-TPM imaging mode, respectively. The laser (Chameleon Vision-S, Coherent, USA; center wavelength: 920 nm) passes through the H-ZF62 glass tube, and subsequently is modulated by the acousto-optic modulator (MT110-B50A1,5-IR-Hk, AA Sa, Orsay Cede, France), and then the laser with the center wavelength in 920 nm is split into two bundles: one is coupled into an HC-920 used in the FIRM-TPM, and the other is coupled into another HC-920 used in the benchtop TPM.

As shown in FIG. 6, in the benchtop TPM mode, collimated laser from the HC-920 is scanned by a pair of galvanometer scanning mirrors A1 (6215H, Cambridge Technology, MA, USA), then passes through a scan lens A2 (LMS05-BB, Thorlabs Inc, New Jersey), a tube lens A3 (Olympus Japan), and a first dichroic lens A4 (DM1, DMLP650R, Thorlabs Inc, New Jersey, USA), and then is finally transferred to a back focal plane (BFP) of an objective A5 (CFI Apo 40XW NIR, Nikon, Japan; 40xNA 0.8, working distance: 3.5 mm). A fluorescence emission signal from the objective A5 is reflected by the first dichroic lens A4, passes through a collecting lens A6 (LA1213-A, Thorlabs Inc, New Jersey, USA), a second dichroic lens A7 (DM2, #87-284, Edmund Optics Inc., Barrington, USA, reflection band: 375-393, 466-490, and 546-565 nm; transmission band: 420-460, 510-531, 590-624, and 677-725 nm), passes through a condensing lens A8 (ACL25416U-A, Thorlabs Inc, New Jersey, USA), and is finally detected using a photomultiplier tube A9 (GaAsP PMT 7422P-40, Hamamatsu, Japan). In order to achieve the same excitation NA as the FIRM-TPM (0.685), a beam of the excitation laser beam at the back focal plane (BFP) of the objective A5 is dimensioned as:

$$(2 \times 0.685 \times 200)(mm)/40 = 6.85(mm)$$

where 200 mm is the EFL of the matched tube lens A3 for the objective A5, and 40 is magnification of the objective A5.

As shown in FIG. 7 and FIG. 8, in the wide-field imaging mode and the FIRM-TPM imaging mode, the miniature probe 2 provided in the present invention is coupled onto a custom-made micro triaxial manual microscopic manipulator (Sigma Koki, Tokyo, JAPAN). An objective image plane in the miniature probe 2 is a focal plane of an air objective (Plan Apo NIR 5X, Mitutoyo, Japan; 5x, NA is 0.14, and a working distance is 37.5 mm). A 4-port objective turret (OT1, Thorlabs Inc, New Jersey, USA) with high-precision bi-directional repeatability ($\pm 2.5\ \mu m$) is used to switch between the miniature probe 2 and a 40x Nikon objective. By adjusting threads of a lens barrel before the Nikon objective and the miniature probe 2, the miniature probe 2 and the Nikon objective can be provided to have a same focal plane in the present invention.

As shown in FIG. 7, in a miniature wide FOV fluorescence structure, a fluorescence luminaire (X-Cite 120Q, Excelitas Technologies, MA, USA): 35 nm) having a blue light filter (MF475-35, Thorlabs Inc, New Jersey, USA; a center wavelength of 475 nm, and a band width of 40 nm) is used as a light source B1. The first dichroic lens A4 in the benchtop TPM configuration is changed into a dichroic lens B2 (DM3, #87-284, Edmund Optics Inc., Barrington, NJ, USA; reflection band: 375-393, 466-490, and 546-565 nm; the transmission band includes: 420-460, 510-531, 590-624, and 677-725 nm), so as to reflect illumination light to the miniature probe 2, and transmit the fluorescence emission signal from the miniature probe 2 to a scientific CMOS camera B3 (8050M-GE, Thorlabs Inc, New Jersey, USA). A similar mean power is used behind a target in imaging in the benchtop TPM and the FIRM-TPM configuration, generally 10-25 mW. Due to differences between single-photon excitation absorption cross-section and two-photon excitation absorption cross-section, a mean power used in the single-photon imaging is usually far lower than a mean power in the two-photon imaging. Therefore, 100-500 $\mu W$ illumination (behind the miniature objective) is used in the wide-field fluorescence microscope configuration in the present invention, similar to 170-600 $\mu W$ illumination used in a previous report.

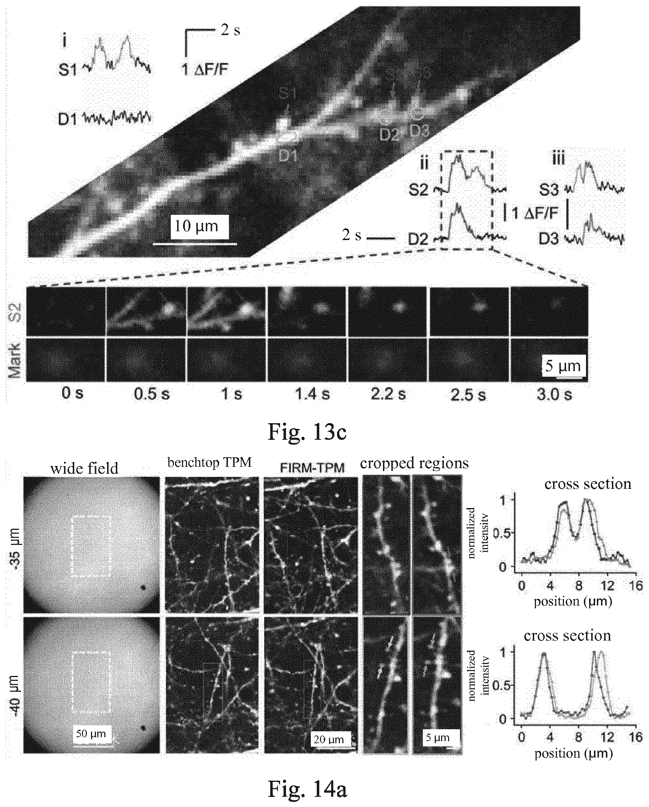

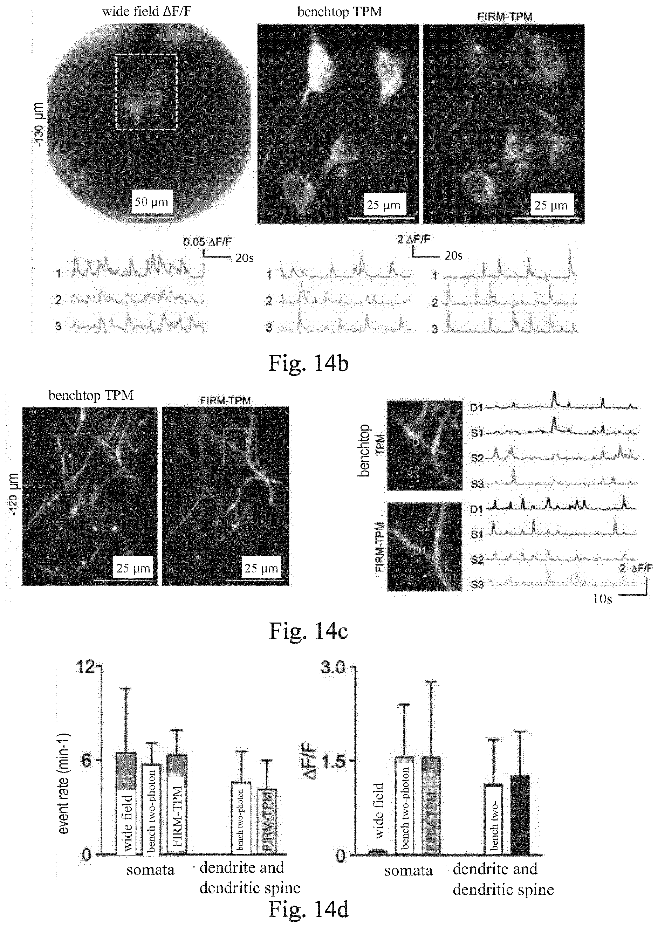

Left-side images in FIG. 14a are 3D morphological imaging of neuronal dendrites and dendritic spines in Thy1-GFP transgenic live specimen brain, obtaining images of substantially the same focal plane under 1-s total exposure (a mean value of 8 frames of 8 Hz of the FIRM-TPM and the miniature wide FOV microscope, and a mean value of 2 frames of 2 Hz of the benchtop TPM), and right-side curves in FIG. 14a show cross-sectional profiles of two pairs of adjacent dendritic spines in cropped regions shown in the left-side images. FIG. 14b shows images of a neuronal-somata-enriched plane (-130 $\mu m$) in PFC of the live specimen expressing GCaMP-6f, wherein upper pictures are 30-second average images of a same ROI, the miniature wide-field image is displayed as normalized $\Delta F/F$ (refer to online methods); lower wavy lines show time course of $Ca^{2+}$ changes (duration: 100 s) in three selected neurons (marked with numerals in the images above). Left-side images in FIG. 14c show $Ca^{2+}$ signals of dendrites and spines (focal plane -120 $\mu m$) from the same live specimen (FIG. 14b), and as distinguishable dendrite signal is lacked, no image recorded by the miniature wide FOV microscope is shown. An upper right image in FIG. 14c shows imaging of an annotated dendritic shaft (D1) and dendritic spines (S1, S2, S3) captured using the benchtop TPM and a $Ca^{2+}$ signal (duration: 100 s), and a lower right image in FIG. 14c shows imaging of the annotated dendritic shaft (D1) and the dendritic spines (S1, S2, S3) (left) captured with the FIRM and a $Ca^{2+}$ signal (duration: 100 s). FIG. 14d shows average frequencies and amplitudes of the $Ca^{2+}$ signals imaged in different configurations, Data are from the same set of neuronal somata (n=4), dendrites (n=8) and dendritic spines (n=6).

For morphological imaging, 3D image data (FIG. 14a) from a V1 region (130x130 $\mu m^2$, from a surface to 60 $\mu m$ below) in a fixed brain from a Thy1-GFP transgenic live specimen is acquired in the present invention. Both the FIRM-TPM and the benchtop TPM exhibit identical contrast and resolution in dendrite imaging (FIG. 14a). However, little structural detail is discernible in the miniature wide-field imaging mode, mainly because of strong background signals from out-of-focus tissues. To compare the functional imaging, a $Ca^{2+}$ indicator GCaMP-6f is expressed in live specimen prefrontal cortical neurons by adeno-associated virus-based infection in the present invention, and calcium imaging is performed in a head-fixed awake live specimen (FIG. 14b to FIG. 14d). Two focal planes are chosen to visualize activity from somata (130 $\mu m$ below the surface) or dendrites and dendritic spines (120 $\mu m$ below the surface). After image normalization and contrast enhancement ($\Delta F/F$), miniature wide-field imaging resolves somatic $Ca^{2+}$ signals at a similar frequency, whereas their amplitudes are approximately an order of magnitude lower ($\Delta F/F$ of 5-10%) than those measured with the benchtop TPM or the FIRM-TPM ($\Delta F/F$ of about 150%) (FIG. 14d). Notably, most of the $Ca^{2+}$ signals that originate from dendritic structures go undetected in such imaging configuration (FIG. 14b and FIG. 14d). Taken together, test of these baselines proves that the FIRM-TPM in the present invention attains performance comparable to that of the benchtop TPM, while outperforming the miniature wide-field microscope in resolving the structure and functional activity of dendrites and dendritic spines in awake animals.

The fast micro-electromechanical scanner 22 used in the FIRM-TPM exhibits greater linear voltage-tilt response and controllability than the resonance scanner used in high-speed benchtop TPMs. Thus, it confers greater flexibility in imaging thereof, such that a single system can achieve fast raster-scan imaging, random-scan imaging of arbitrarily designated regions of interest (ROIs), and ultrafast line scan imaging. In particular, Random-scan imaging at 128 Hz aids in resolving subtle temporal substructures of the $Ca^{2+}$ signals, and may endow the mTPM with the capability of precise optogenetics manipulation in freely behaving animals. A 10-kHz line-scanning imaging capability is of vital importance for fast observation of action-potential with genetically encoded voltage indicator.

Dendritic spine activities are elementary events of neuron information processing, as manifested by the activation of different dendritic spines on single nerve cells in head-fixed animals by visual stimulations of different orientations or by auditory stimulations of different intensities and frequencies under benchtop TPMs. Although ensembles of activities from hundreds of neurons acquired by the miniature wide-field microscopes can help to probe neuronal-network coding properties under different behavioral conditions, the FIRM-TPM in the present invention provides an option of a more powerful tool, to uncover the spatiotemporal characteristics of more fundamental neural encoding unit in behavioral paradigms unable to be realized in head-fixed animals.

In sum, the newly developed FIRM-TPM has achieved high spatiotemporal resolution, good mechanical stability, and effective excitation of prevalent indicators. With use of the new generation mTPM, the present invention achieves the goal of imaging at the single-dendritic-spine level in freely moving animals over a prolonged time period that is always longed for by scientists. More improvements and expansions in the future can be anticipated. FOV and penetration depth can be further expanded using a correctly designed miniature high-NA objective. Deep brain imaging can be realized by directly inserting the miniature objective into the brain, or by embedding the GRIN lens in the brain, similarly to previously described methods. For larger animals, for example, rats or marmosets, it would also be possible to mount multiple FIRM-TPM s to the skull to address questions related to long-range structural and functional connections in the brain. It is predicted in the present invention that the FIRM-TPM will be proved to be an important tool for neuroscientists and biomedical scientists to explore health and disease mechanisms in animal bodies.

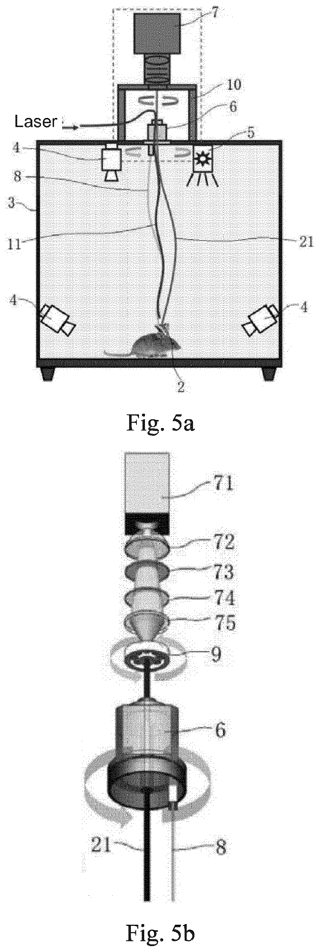

As shown in FIG. 5a, the present invention further provides a live specimen behavior imaging system, wherein the live specimen behavior imaging system includes a case 3, a femtosecond pulse laser, a femtosecond pulse laser modulator 1, a miniature probe 2, a data collecting assembly 7, and a wiring installation assembly, wherein the femtosecond pulse laser is configured to produce laser with a wavelength of 920 nm. The femtosecond pulse laser modulator 1 is configured to receive the laser output by the femtosecond pulse laser, amplify a transmission power of the laser to a predetermined value thereof, compensate for pulse-broadening of the laser through prechirp, and then output the compensated laser. The miniature probe 2 is configured to be mounted on a body of the live specimen, has an input end connected to an output end of the femtosecond pulse laser through a laser input fiber in the femtosecond pulse laser modulator, is configured to receive the laser output by the femtosecond pulse laser modulator, which laser scans tissues inside the live specimen to excite the live specimen to produce a fluorescence signal, and is configured to receive the fluorescence signal output by the scan imaging portion, and output the fluorescence signal. The femtosecond pulse laser, the femtosecond pulse laser modulator 1, and the miniature probe 2 are all introduced in detail in the preceding text, and will not be described redundantly herein.

The case 3 provides a restricted space for free movement of the live specimen, wherein the restricted space is much larger than a size of the live specimen, and can provide a big enough space for free activities of the live specimen. The data collecting assembly 7 is mounted on the case 3. The data collecting assembly 7 has an input end connected to an output end of the miniature probe 2 through a fluorescence output fiber 21, and is configured to collect the fluorescence signal output by the miniature probe 2. The laser input fiber 11 and the fluorescence output fiber 21 are mounted on the case 3 through the wiring installation assembly in a manner of being rotatable at will relative to the case 3. Through the wiring installation assembly, entanglement among the fluorescence output fiber 21, the laser input fiber 11, and other electrical wires 8 can be prevented, providing an advantageous condition for the free activities of the live specimen.

In one embodiment, the data collecting assembly 7 provides a detection light path, which detection light path includes coaxially arranged photomultiplier tube 71, condenser 72, emission filter 73, short-pass filter 74, and collecting lens 75, wherein the photomultiplier tube 71 is configured to convert an optical signal into an electrical signal and then output the electrical signal, the condenser 72 is configured to converge the fluorescence signal transmitted by the fluorescence output fiber 21, the emission filter 73 is configured to filter out laser with a wavelength of 920 nm, the short-pass filter 74 is configured to filter out stray light other than signal light, the fluorescence output fiber 21 is coaxial with the collecting lens 75, the fluorescence output fiber 21 is located on a focal plane of the collecting lens 75, and the collecting lens 75 is configured to more sufficiently collect the fluorescence signal into the photomultiplier tube 71.

Preferably, the wiring installation assembly includes an electrical rotary joint 6 and a holder 10, wherein the electrical rotary joint 6 is provided penetrating through a top portion of the case 3, and has one end coupled to an external power supply, and the other end coupled to the electrical wires 8, such that the external power supply and the electrical wires 8 can be electrically coupled. The holder 10 is covered over the electrical rotary joint 6 located in the top portion of the case 3, the laser input fiber 11 and the fluorescence output fiber 21 pass through an outer housing of the electrical rotary joint 6, and the fluorescence output fiber 21 is in optical-signal-connection with the collecting lens 75 in the data collecting assembly 7. The fluorescence output fiber 21 is connected to the data collecting assembly 7 through a bearing 9, thus the electrical rotary joint 6 and the bearing 9 allow the fluorescence output fiber 21 and the electrical wires 8 to rotate independently without altering the detection light path, which design prevents entanglement of connecting lines when the live specimen is freely exploring its environment, and greatly improves the stability of the miniature probe 2, even when the live specimen is engaged in intense physical activity, so as to minimize twisting and entanglement of lines when the animal is freely exploring.

In one embodiment, the freely moving live specimen behavior imaging system further includes the electrical wires 8 supplying power to the micro-electromechanical scanner 22. The electrical wires 8 are mounted on the case through the wiring installation assembly in a manner of being rotatable at will relative to the case, thereby ensuring normal electricity supply by the electrical wires 8, and also being capable of avoiding entanglement of the electrical wires 8 with the laser input fiber 11 and the fluorescence output fiber 21, so as to minimize twisting and entanglement of lines when the animal is freely exploring.

In one embodiment, the freely moving live specimen behavior imaging system further includes multiple cameras 4, wherein the cameras 4 are all mounted on an inner wall of the case 3, for example, mounted on side faces and a top face of the case 3 shown in FIG. 2, for shooting and recording behaviors in a freely moving process of the live specimen from different angles.

In one embodiment, the freely moving live specimen behavior imaging system further includes an illumination lamp 5, wherein the illumination lamp 5 is mounted on an inner wall of the case 3 for lighting an inner chamber of the case 3.

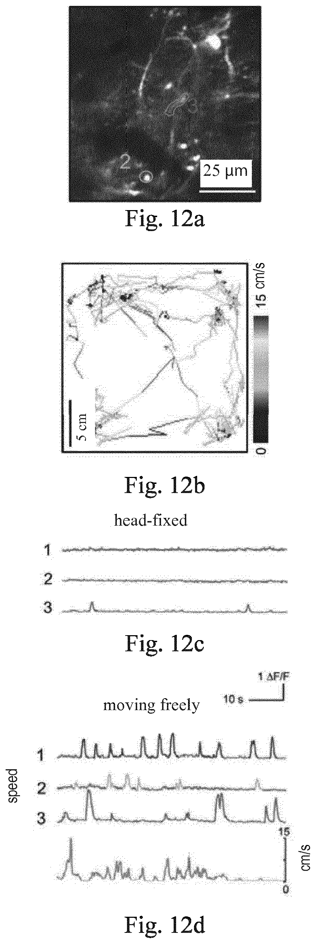

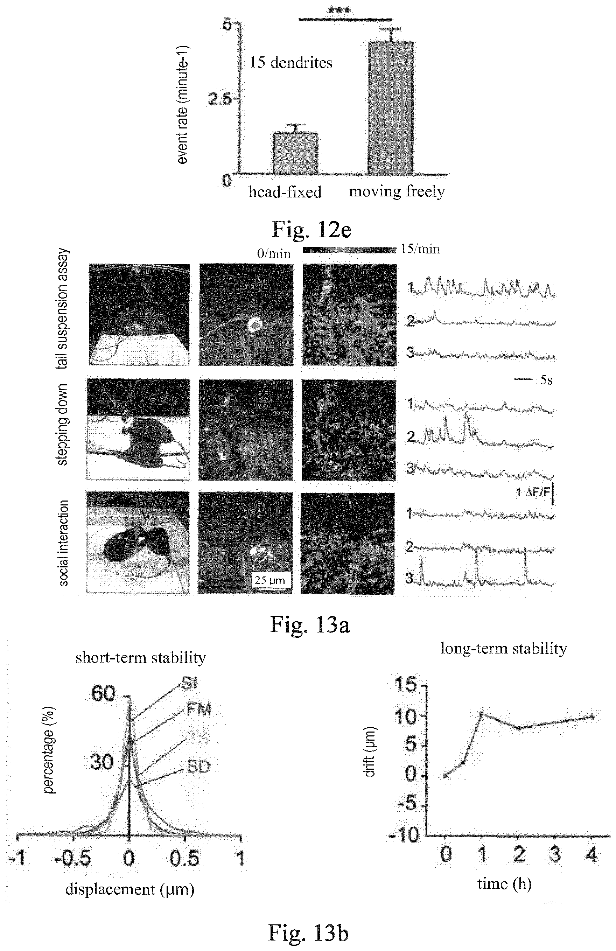

As shown in FIG. 12a to FIG. 12e, FIG. 12a shows an average image of 50 FIRM-TPM (the miniature two-photon microscopic imaging device in the present invention) images of V-1 cortical L2/3 neuron, in which image dendrites 1, 2, and 3 are plotted. FIG. 12b shows a two-dimensional trajectory of the live specimen in an arena (duration: 100 s), wherein the trajectory is calculated according to video recording captured by the cameras on the top, and moving speeds are encoded with colors. FIG. 12c and FIG. 12d respectively show time courses (duration: 100 s) of $Ca^{2+}$ signals from the three dendrites (plotted in FIG. 12a) under head-fixed (FIG. 12c) and free moving conditions (FIG. 12d), and time-dependent variation of the live specimen speed is also shown at the bottom. FIG. 12e shows average frequencies of all dendritic $Ca^{2+}$ transients under head-fixed and free moving conditions; data are represented by an average value $\pm$ s.e.m., with n=15 dendrites. ***p<0.001, and paired t test. From the above experimental data, we can find a significant increase in the visual cortical neuronal dendritic activity when the live specimen is freely exploring in the dark (the trajectory is shown in FIG. 12a to FIG. 12e), relative to the activity when the same live specimen is head-fixed (FIG. 12a to FIG. 12e).

As shown in FIG. 13a to FIG. 13c, FIG. 13a shows imaging of neuronal activities in visual cortex within three different behavioral paradigms: tail suspension assay, stepping down from an elevated stage, and social interaction. Images in a first column on the leftmost side: snapshots of the live specimen engaging in different behaviors captured by two cameras on side faces of the behavioral device; images in a middle second column: FIRM-TPM imaging of GCaMP6f-labeled neuronal somata, dendrites, and dendritic spines; images in a right third column: images of corresponding calcium-mobilization distribution (duration: 100 s); images in a fourth column on the rightmost side: $Ca^{2+}$ signals (duration: 50 s) from three ROIs indicated in the FIRM-TPM images. FIG. 13b shows short- and long-term stability of the FIRM-TPM imaging, wherein a left-side curve in FIG. 13b shows lateral displacement distribution in each behavioral paradigm on the basis of frame-to-frame cross-correlation analysis, wherein FM represents free moving; TS represents tail suspension; SD represents stepping down from an elevated stage; and SI represents social interaction. A right-side curve in FIG. 13b shows drift of FOV in 4-h experiment session.

This result is in agreement with findings from multisite electrical probe recording of enhanced visual cortical neuronal activity by movement of the animal. Next, to demonstrate the robustness of the FIRM-TPM, in the present invention, the neuronal activity is observed by performing imaging of primary visual cortex at 40 Hz over an FOV of $130 \times 130$ $\mu m^2$ in the live specimen that is sequentially tested in three behavioral paradigms including tail suspension, stepping down from an elevated stage, and social interaction. None of these paradigms can be realized in a head-fixed test strategy, and the entire test protocol lasts approximately 4 h. It is noteworthy that activity from different somata, dendrites, and dendritic spines still can be stably observed even when the live specimen is struggling in the tail suspension assay, stepping down from an elevated stage, or interacting with its sibling. Different behavioral paradigms appear to be associated with nervous activities in different regions of the same V1 cortex (FIG. 13a).

In order to quantify the short- and long-term stability of the miniature two-photon microscope provided in the present invention, lateral displacements are analyzed through frame-by-frame cross-correlation and session-to-session drift of the FOV (FIG. 13b). It is found in the present invention that within 2000 consecutive frames, the average frame-to-frame displacement is the largest during the stepping-down paradigm ($0.19\pm0.09$ $\mu m$; 75% within 0.24 $\mu m$) and remains less than half of the size of a pixel. During the entire 4-h test, the overall drift of the FOV of the FIRM-TPM is <10 $\mu m$, and most of the drift occurs during a first hour of intense activity in the tail suspension experiment. Notably, because a relatively high image collecting speed decreases in-frame blurring, this level of motion artifact does not preclude the present invention from imaging dendritic spine structure and activity. In order to demonstrate the imaging capability of the miniature two-photon microscope provided in the present invention for activities of a single synapse, by tracking Ca$^{2+}$ activities on the dendritic spine and parental dendritic shaft thereof, the present invention further analyzes relation between global and local activities. To sum up, the miniature two-photon microscope provided in the present invention can stably observe the dendrite and dendritic spine activities in freely behaving animals in a natural physiological environment.

The present invention further provides a miniature two-photon microscopic imaging method, wherein the miniature two-photon microscopic imaging method includes:

Step 1, choosing an region to be imaged: fixing a live specimen, and choosing the region to be imaged from the live specimen using a benchtop TPM, wherein in Step 1, an imaging platform as shown in FIG. 6 is needed, and the region to be imaged is chosen from the live specimen in a benchtop TPM mode, that is to say, using the benchtop TMP, with following specific operation: fixing the live specimen with a body retainer, so as to confirm a viral infection region of head of the live specimen through the benchtop TPM;

Step 2, collecting a fluorescence signal: mounting a miniature probe connected to a laser input fiber on the live specimen, and releasing the live specimen, to detect the fluorescence signal output from the region to be imaged chosen in Step 1, and completing imaging of a tissue plane inside the live specimen, wherein specific operations in Step 2 are as follows:

First, the miniature probe 2 provided in the present invention is bonded onto a holder on head skull of the live specimen, wherein the holder has been fixed on the head skull of the live specimen during craniotomy preparation, and then the miniature probe 2 is reinforced with dental cement onto the holder on the head skull of the live specimen.

Thereafter, 1.5% low-melting-point agarose is applied to cover an exposed brain tissue region. This process substantially dampens the relative movement between the probe and brain. In order to realize dyeing of lots of neurons in random access, a process of labelling the neurons is as follows: an artificial cerebrospinal fluid (Sigma-Aldrich, Shanghai, China; 125 mM NaCl, 4.5 mM KCl, 26 mM NaHCO$_3$, 1.25 mM NaH$_2$PO$_4$, 2 mM CaCl$_2$, 1 mM MgCl$_2$, 20 mM glucose, pH of 7.4 when bubbled with 95% O$_2$ and 5% CO$_2$) is used in the present invention to cover the exposed brain region. 50 $\mu$g of Cal-520 AM (AAT Bioquest. CA, USA) is dissolved in 4$\mu$l DMSO (with 20% Pluronic F-127), and diluted to 500 $\mu$M with an artificial diluent (150 mM NaCl, 2.5 mM KCl, 10 mM Hepes, pH 7.432). Borosilicate pipettes of 2-3 M$\Omega$ resistance is used to inject the Ca$^{2+}$ dye into layer 2/3 of the primary visual cortex by application of a pressure pulse (1 min, 400 mbar). After one hour, SR-101 is dropwise applied to the cortical surface to identify astrocytes. The live specimen is head-fixed, and imaging is performed with the FIRM-TOP in a random-access mode.

Finally, the live specimen is released from the retainer, and placed in the case 3 of small animal imaging system in the above example, and tail suspension experiment assay or sliding down experiment assay (cylindrically platform, 5 cm$^2$, 5 cm high) and social interaction (square activity space of 30×30 cm$^2$) are conducted for the live specimen using the miniature probe 2.

Step 3, processing the fluorescence signal, to acquire a three-dimensional image of the live specimen's free activity.

Before the above step, following Step 4 is further included: preparing the live specimen, specifically including:

Step 41, choosing the live specimen: C57BL/6 wild-type and Thy-1-GFP transgenic live specimens (8-26 postnatal weeks) are used in the experiments. All procedures are approved by the Peking University Animal Use and Care Committee and complies with the standards of the Association for Assessment and Accreditation of Laboratory Animal Care, including the animal breeding and experimental manipulation. The Thy-1-GFP transgenic live specimen is anesthetized and perfused with 0.9% saline followed by 4% paraformaldehyde, and the brain is removed, and stays overnight in 4% paraformaldehyde in PBS at 4 ºC.

Step 42, craniotomy for cortical imaging *in vivo*: C57BU6 wild-type live specimens are used in these experiments. Briefly, each live specimen is anesthetized by inhalation of 1-1.5% isoflurane in pure O$_2$, and placed on a stereotaxic frame (68025, RWD, Shenzhen, China). Meanwhile, a warming plate (37.5-38 ºC) is used to maintain the normal temperature of the live specimen. After local application of xylocaine and removal of skin and mussels, a small square cranial window (0.5×0.5 mm$^2$) is made with a high speed craniocerebral drill (tip diameter 0.5 mm) and is centered over the of targeted cortex. GCaMP-6f is expressed with recombinant AAV under the calmodulin-dependent kinase II (CaMK II) promoter (serotype 2/9; >2×1013 genome copies per milliliter, produced by the University of Pennsylvania Gene Therapy Program Vector Core. A total of 0.5-0.8 $\mu$l of AAVis slowly injected within 20 min into layer 2/3 (depth of 130-400 $\mu$m) of the targeted cortex of the live specimen with an injector (Nanoliter 2010, World Precision Instruments Sarasota, USA) and glass microelectrodes. After 3 weeks of expression, the live specimens are anesthetized with isoflurane, a homemade holder is attached to the skull with cyano-acrylic gel and the attachment is reinforced with dental cement. A small square cranial window (2.5×2.5 mm$^2$) is made and centered over the targeted cortex using the craniocerebral drill. The dura is carefully removed, and a small glass coverslip (3.5×2.5

mm$^2$) is placed on the craniotomy. After recovery for one week, each live specimen is trained to adapt to head fixation for 30 min per day for 3 days continuously. After the awake live specimens are adapted, Step 1 and Step 2 are operated.

Finally, it needs to be indicated that the embodiments above are merely for describing the technical solutions of the present invention, rather than limiting the same. A person ordinarily skilled in the art should understand that modifications can be made to the technical solutions described in the various preceding embodiments, or equivalent substitutions can be made to some technical features therein; all of such modifications or substitutions will not essentially depart corresponding technical solutions from the spirit and scope of the technical solutions of various embodiments of the present invention.

**Claims**

1. A femtosecond pulse laser modulator, comprising:

    a negative dispersion light path and a positive dispersion light path, wherein
    the negative dispersion light path comprises a laser input fiber, wherein the laser input fiber is configured to transmit laser compensated for through prechirp;
    the positive dispersion light path is located between a femtosecond pulse laser and the negative dispersion light path, and is configured to compensate for, through prechirp, negative dispersion of the laser produced in the laser input fiber.

2. The femtosecond pulse laser modulator according to claim 1, wherein the input fiber is a hollow-core photonic crystal fiber, providing distortion-free transmission for the femtosecond pulse laser with a center wavelength in 920 nm.

3. The femtosecond pulse laser modulator according to claim 2, wherein a fiber core material of the hollow-core photonic crystal fiber is pollution-free silicon, and a fiber core diameter is 8-9 $\mu$m.

4. The femtosecond pulse laser modulator according to claim 1, wherein the positive dispersion light path comprises a dispersion compensation element, and the dispersion compensation element is of a commercial material.

5. The femtosecond pulse laser modulator according to claim 4, wherein the dispersion compensation element is an H-ZF62 glass tube.

6. The femtosecond pulse laser modulator according to claim 4, wherein the positive dispersion light path further comprises an acousto-optic modulator, and the acousto-optic modulator is configured to receive laser compensated for by the dispersion compensation element, adjust laser intensity, and then output the laser to the laser input fiber.

7. The femtosecond pulse laser modulator according to claim 6, wherein the positive dispersion light path further comprises a laser orientation adjustment assembly, which is provided between the dispersion compensation element and the acousto-optic modulator, for adjusting modulation efficiency of the acousto-optic modulator.

8. The femtosecond pulse laser modulator according to claim 6, wherein the positive dispersion light path further comprises a spectroscopic assembly, configured to split the laser modulated by the acousto-optic modulator into at least two bundles, and output the split laser to the laser input fiber.

9. A miniature two-photon microscopic imaging device, comprising:

    a femtosecond pulse laser, configured to produce laser with a center wavelength in 920 nm;
    the femtosecond pulse laser modulator of any one of claims 1-8, configured to receive the laser output by the femtosecond pulse laser, perform pulse amplification, and then output the amplified laser to a miniature probe; and
    the miniature probe, wherein the miniature probe comprises:

        a scan imaging portion, configured to receive the laser output by the femtosecond pulse laser modulator, and scan tissues inside a live specimen using the laser, so as to excite a biological indicator inside the live specimen to produce a fluorescence signal; and

a fluorescence output fiber, which is configured to output the fluorescence signal.

10. The miniature two-photon microscopic imaging device according to claim 9, wherein the scan imaging portion comprises:

a micro-electromechanical scanner, configured to perform two-dimensional scanning for tissues inside the live specimen in a manner of altering magnitude of a laser incident angle through rotation;
an objective, configured to converge the laser from the micro-electromechanical scanner into the live specimen;
a scan lens, which is arranged on a light path between the micro-electromechanical scanner and the objective, configured to convert the laser altered in angle caused by the two-dimensional scanning of the micro-electromechanical scanner into position-altered laser; and
a dichroic mirror, which is provided between the scan lens and the objective, configured to separate the laser from the fluorescence signal.

11. The miniature two-photon microscopic imaging device according to claim 10, wherein the objective has a numerical aperture of 0.8.

12. The miniature two-photon microscopic imaging device according to claim 10, wherein 1.5% low-melting-point agarose is filled in between the objective and the live specimen.

13. The miniature two-photon microscopic imaging device according to claim 10, wherein the objective comprises a thin Fresnel lens.

14. The miniature two-photon microscopic imaging device according to claim 10, wherein the scan imaging portion further comprises a collimator, which is arranged between the laser input fiber and the micro-electromechanical scanner.

15. The miniature two-photon microscopic imaging device according to claim 9, wherein the fluorescence output fiber is a supple fiber bundle.

16. The miniature two-photon microscopic imaging device according to claim 15, wherein the supple fiber bundle is made by fusing two ends of each of 700-900 glass optical fibers while keeping various glass optical fibers loose and separated.

17. A live specimen behavior imaging system, comprising

the miniature two-photon microscopic imaging device according to any one of claims 9-16;
a case, configured to provide a restricted space for free movement of the live specimen;
a wiring installation assembly, through which the laser input fiber and the fluorescence output fiber are mounted on the case in a manner of rotating at will with respect to the case; and
a data collecting assembly, configured to gather the fluorescence signal output by the fluorescence output fiber.

18. The live specimen behavior imaging system according to claim 17, wherein the wiring installation assembly comprises:

an electrical rotary joint, which is provided penetrating through a top portion of the case, the laser input fiber and the fluorescence output fiber passing through an outer housing of the electrical rotary joint;
a holder, which covers the outer housing of the electrical rotary joint, and on which the data collecting assembly is provided; and
a bearing, through which the fluorescence output fiber is connected to the data collecting assembly.

19. The live specimen behavior imaging system according to claim 18, wherein the electrical rotary joint has one end outside the case coupled to an external power supply, and the other end inside the case providing an electrical connection port.

20. A miniature two-photon microscopic imaging method, comprising:

choosing a region to be imaged: fixing a live specimen, and choosing the region to be imaged from the live specimen using a benchtop two-photon microscope; and

collecting a fluorescence signal: mounting a miniature probe of the miniature two-photon microscopic imaging device according to any one of claims 9-16 on the live specimen, and releasing the live specimen, to collect a fluorescence signal output from the region to be imaged.

Fig. 1

Fig. 2

Fig. 3

Fig. 4

Fig. 5a

Fig. 5b

920-nm femtosecond laser

Fig. 6

excitation light
emission light
imaging plate
object plate

FIRM-TPM

Fig. 7

11
920-nm laser
emission
light
73 71
2 21 75

Fig. 8

Fig. 9a

Fig. 9b

Fig. 9c

Fig. 9d

Fig. 9e

Fig. 9f

Fig. 10a

Fig. 10b

Fig. 10c

Fig. 11a

Fig. 11b

Fig. 11c

Fig.11d

Fig. 12a

Fig. 12b

head-fixed

Fig. 12c

moving freely

Fig. 12d

Fig. 12e

Fig. 13a

Fig. 13b

Fig. 13c

Fig. 14a

Fig. 14b

Fig. 14c

Fig. 14d

# INTERNATIONAL SEARCH REPORT

International application No.

PCT/CN2018/076305

## A. CLASSIFICATION OF SUBJECT MATTER

H01S 3/00 (2006.01) i; A61B 5/00 (2006.01) i

According to International Patent Classification (IPC) or to both national classification and IPC

## B. FIELDS SEARCHED

Minimum documentation searched (classification system followed by classification symbols)

H01S 3; A61B 5

Documentation searched other than minimum documentation to the extent that such documents are included in the fields searched

Electronic data base consulted during the international search (name of data base and, where practicable, search terms used)

CNABS; CNTXT; CNKI; VEN; USTXT: 飞秒, 脉冲, 激光, 正色散, 负色散, 空心光子晶体, 直径, 光纤, 探头, 荧光, 扫描, 活体, Femtosecond, pulse, laser, negative, fiber, optical, dispersion, photon, scanning

## C. DOCUMENTS CONSIDERED TO BE RELEVANT

| Category* | Citation of document, with indication, where appropriate, of the relevant passages | Relevant to claim No. |
|---|---|---|
| PX | CN 107069391 A (PEKING UNIVERSITY), 18 August 2017 (18.08.2017), description, paragraphs [0024]-[0131], and figures 1-14d | 1-20 |
| A | CN 102621765 A (INSTITUTE OF PHYSICS CHINESE, ACADEMY OF SCIENCES), 01 August 2012 (01.08.2012), description, paragraphs [0037]-[0055], and figures 1-7 | 1-20 |
| A | CN 104283097 A (SHANGHAI ROI OPTOELECTRONICS TECHNOLOGY CO. LTD.), 14 January 2015 (14.01.2015), description, paragraphs [0008]-[0038], and figures 1-5 | 1-20 |

☐ Further documents are listed in the continuation of Box C.      ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 April 2018 | 03 May 2018 |

| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br><br>YU, Lifei<br><br>Telephone No. 86-(512)-88997435 |

Form PCT/ISA/210 (second sheet) (January 2015)

## INTERNATIONAL SEARCH REPORT
### Information on patent family members

International application No.

PCT/CN2018/076305

| Patent Documents referred in the Report | Publication Date | Patent Family | Publication Date |
|---|---|---|---|
| CN 107069391 A | 18 August 2017 | None | |
| CN 102621765 A | 01 August 2012 | None | |
| CN 104283097 A | 14 January 2015 | None | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Nanoliter. World Precision Instruments Sarasota, 2010